# EUROPEAN PATENT SPECIFICATION

(11) **EP 2 723 878 B1**
(45) Date of publication and mention of the grant of the patent: **20.05.2020**
(21) Application number: 12732810.2
(22) Date of filing: 20.06.2012
(51) Int. Cl.: C12P 13/02, C12N 1/20

(54) **VITAMIN B5 PRODUCTION**
HERSTELLUNG VON VITAMIN B5
PRODUCTION DE VITAMINE B5

(30) Priority: 21.06.2011 CH 10602011
(43) Date of publication of application: 30.04.2014
(73) Proprietor: DSM IP Assets B.V., 6411 TE Heerlen (NL)
(72) Inventor: GRUNER, Ines, CH-4002 Basel (CH); JEUDE, Markus, CH-4002 Basel (CH); PRAGAI, Zoltan, CH-4002 Basel (CH)
(74) Representative: Seibel-Thomsen, Nadja
(86) International application number: PCT/EP2012/061890
(87) International publication number: WO 2012/175575

(56) References cited:
- WO-A1-2007/065602
- WO-A2-02/057474
- WO-A2-2004/113510
- WO-A2-2008/148575
- US-A1- 2004 048 344
- JEUDE M ET AL: "Fed-batch mode in shake flasks by slow-release technique", BIOTECHNOLOGY AND BIOENGINEERING, WILEY & SONS, HOBOKEN, NJ, US, vol. 95, no. 3, 1 October 2006 (2006-10-01), pages 433-445, XP002497978, ISSN: 0006-3592, DOI: 10.1002/BIT.21012 [retrieved on 2006-05-30]
- PROvendis GmbH: "Effizient nach den richtigen Bakterien oder Zellen suchen", Pressemitteilung 05-11 , 22 August 2005 (2005-08-22), XP002692558, Retrieved from the Internet: URL:http://www.technologieallianz.de/webte mp/FeedBeads449a38ae.pdf [retrieved on 2013-02-22]
- Kuhner shaker: "FeedBeads", Flyer FeedBeads , XP002692559, Retrieved from the Internet: URL:http://www.kuhner.com/de/downloads.htm l [retrieved on 2013-02-22]
- PANULA-PERÃ LÃ JOHANNA ET AL: "Enzyme controlled glucose auto-delivery for high cell density cultivations in microplates and shake flasks", MICROBIAL CELL FACTORIES, BIOMED CENTRAL, vol. 7, no. 1, 18 November 2008 (2008-11-18), page 31, XP021043554, ISSN: 1475-2859, DOI: 10.1186/1475-2859-7-31
- SEVASTSYANOVICH Y ET AL: "Recombinant protein production: a comparative view on host physiology", NEW BIOTECHNOLOGY, ELSEVIER BV, NL, vol. 25, no. 4, 1 April 2009 (2009-04-01), pages 175-180, XP026059202, ISSN: 1871-6784, DOI: 10.1016/J.NBT.2009.01.006 [retrieved on 2009-01-22]

## Description

The present invention relates to an improved production process for pantothenic acid (vitamin B5) using a *Bacillus subtilis* strain, said strain of Bacillus subtilis comprising a deregulation in the biosynthesis of leucine, valine or isoleucine and wherein at least one pantothenate biosynthetic gene is overexpressed, in a medium containing 50 mg/l yeast extract and 10 g/l or less of a quickly metabolized sugar which is slowly released into the medium. This medium is particularly useful for high throughput (HTP) screening of *Bacillus subtilis* mutants.

Pantothenate is a member of the B complex of vitamins and is a nutritional requirement for mammals including humans, e.g., from food sources, as a water-soluble vitamin supplement or as a feed additive. In cells, pantothenate is used primarily for the biosynthesis of coenzyme A and acyl carrier protein. These essential coenzymes function in the metabolism of acyl moieties, which form thioesters with the sulfhydryl group of the 4'-phosphopantethein portion of these molecules.

Pantothenate has been synthesized conventionally via chemical synthesis from bulk chemicals. However, the substrates required for chemical synthesis are expensive and the racemic intermediates have to be optically resolved. Accordingly, bacterial or microbial systems have been employed that produce enzymes useful in pantothenate biosynthesis processes. In particular, bioconversion processes have been evaluated as a means of favoring production of preferred isomer of pantothenic acid. Moreover, methods of direct microbial synthesis have recently been examined as a means of facilitating D-pantothenate production.

The genes involved in biosynthesis of pantothenic acid as well as methods for fermentative production of pantothenic acid are known (see e.g. WO 01/21772, WO 02/057474, WO 02/061108 or Ullman's Encyclopedia of Industrial Chemistry, 7th Edition, 2007, Chapter Vitamins).

A method of producing pantothenate or pantoate by culturing genetically modified microorganisms of the genus *Bacillus,* particularly *B*. *subtilis,* in which at least one enzyme selected from the group consisting of PanB (ketopantoate hydroxymethyl transferase), PanC (pantothenate synthetase), PanD (aspartate-α-decarboxylase) and PanE (ketopantoate reductase) is overexpressed is known (WO 01/21772).

A key enzyme in biosynthesis of pantothenic acid is PanB. This enzyme catalyzes the conversion of α-ketoisovalerate to α-ketopantoate; α-ketopantoate in turn is converted to pantoate via the NADPH-dependent PanE reaction. It has been found that this step forms a serious bottleneck in the biosynthesis of pantothenate, *i.e.* is rate-limiting for pantothenate biosynthesis.

Besides genetic engineering of the production strains, there is an ongoing need in optimization of the culture conditions, including but not limited to the use of carbon source, buffer system, pH, oxygen content or the use of growing or resting cells. To achieve higher production rates of the production strains to use them for an industrial production process, this optimization also includes the screening methods, such as *e.g.* high throughput screening, for more powerful production strains.

Surprisingly, it has now been found out that the use of a low amount of a quickly metabolized sugar as carbon source in the (culture/production) medium which is slowly released into the medium leads to increased production yield of pantothenate on (consumed) sugar. In particular, the medium may contain a low amount of glucose combined with higher amount of raffinose. It turned out that the new medium composition is in particular useful for screening of pantothenate high producing strains. Preferred is the use of a medium wherein the release of sugar, preferably glucose, is about 10 g/l after 48 hours for high throughput screening of pantothenate producing strains of *Bacillus subtilis.* Compared to the known culture media used so far (standard medium), the increased production of pantothenate is proportional to the engineering level of the production strain. Using the new medium in a traditional shake flask experiment with cultivation for 48 hours according to the present invention, the pH stayed at a constant level during the whole time and all sugar, *e.g.* glucose and raffinose, was consumed.

In particular, it is an object of the present invention to provide a pantothenate production process wherein strains of *Bacillus subtilis,* are cultivated in a medium wherein the starting concentration of sugar is 10 g/l or less with a slow release of said sugar into the medium. In particular useful for the present invention is a medium, wherein the release of sugar, preferably glucose, is about 10 g/l after 48 hours. Preferably, the starting concentration of quickly metabolized sugars, such as *e.g.* glucose, is 10 g/l or less, preferably 8, 4, 2, 1 g/l or less. In a particular embodiment, the culture medium comprises a mix of glucose (mimicking the batch phase of the fermentation cultivation) and raffinose (mimicking the feed phase of the fermentation cultivation), in a ratio range of 1:2 to 1:20, such as 1:4, 1:5, 1:6, 1: 8, 1:9, 1:10, 1:12, 1:15, preferably in a ratio of 1:9, such as e.g. 1 g/l glucose and 9 g/l raffinose as starting concentration. The glucose concentration may vary from 0 to 10 g/l. Preferably, the glucose concentration is from 0 to 4 g/l.

The glucose may be replaced by other quickly metabolized sugars such as e.g. fructose, ribose or sucrose. The raffinose may be replaced by other oligosaccharides. Thus, the medium according to the present invention can comprise a mixture of glucose, fructose, ribose or sucrose with oligosaccharides such as sucrose, maltose, cyclodextrins, maltotriose or lactose, preferably in the ratios mentioned above for glucose to raffinose, in particular in the ratio of 1:9, wherein the sugar is slowly released into the medium.

Thus, the present invention is directed to a process for the production of pantothenate, wherein a strain of *Bacillus subtilis,* said strain comprising a deregulation in the biosynthesis of leucine, valine or isoleucine and wherein at least one pantothenate biosynthetic gene is overexpressed, is cultivated in a medium comprising a sugar selected from fructose, ribose, sucrose or glucose and wherein the starting concentration of the sugar selected from fructose, ribose, sucrose or glucose is 10 g/l or less supplemented with 50 mg/l yeast extract and wherein the sugar is slowly released into the medium, *i.e.* the release of said sugar is 10 g/l after 48 hours. Optionally, the pantothenate is recovered from the medium. Preferably, the sugar is glucose, but might also be selected from fructose, ribose or sucrose. All sugar is consumed after a cultivation process of 48 hours according to the present invention.

In one particular embodiment, the sugar is released from polymer particles enabling a slow release of the sugar into the medium as defined above (*i.e*. mimicking a fed-batch cultivation). This can be achieved by the use of the FeedBead® system (Adolf Kühner AG, 4127 Birsfelden, Schweiz), wherein FeedBeads are selected such that a glucose release of 10 g/l is obtained after 48 hours of cultivation. In one embodiment, said slow-release systems for sugars may replace furthermore the quickly metabolized sugars mentioned above, such as e.g. glucose, fructose, ribose or sucrose. Preferably, such system could furthermore replace the raffinose in the medium or the other useful oligosaccharides mentioned above. Most preferably, the medium comprises sugar containing polymer particles including, but not limited to, FeedBeads as the only sugar, wherein a slow release into the medium according to the present invention is achieved.

The media as used for the performance of the present invention are based on the one described as RSM in WO 2008/148575 (Example 2), herein further defined as SRG, whereby preferably the glucose and/or raffinose is replaced by FeedBeads. The medium according to the present invention may be with or without citrate, whereby the original medium (SRG) comprises 0.64 g/l citrate. Furthermore, the amount of yeast extract - which in the original SRG medium is 1 g/l - is limited to 50 mg/l.

Thus, the present invention is also directed to biosynthesis of pantothenic acid with a microorganism selected from *Bacillus subtilis,* said strain of *Bacillus subtilis* comprising a deregulation in the biosynthesis of leucine, valine or isoleucine and wherein at least one pantothenate biosynthetic gene is overexpressed, wherein the amount of sugar, citrate and yeast extract in the (production) medium are as shown in Table 1 (see SRG-Y, SRG-CY; SRG-FB-Y, SRG-FB-CY). These media are also useful for HTP screening of B5 high production strains of *Bacillus subtilis.*

As used herein, the term "pantothenic acid" includes pantothenic acid, derivatives thereof such as salts or esters thereof, *i.e.* pantothenate, in particular calcium pantothenate. The terms "pantothenic acid", "pantothenate" and "vitamin B5" are used interchangeably herein.

As used herein, the pantothenate production strain is selected from *Bacillus subtilis* (Zeigler and Perkins, 2008, Practical Handbook of Microbiology", Second Edition (E. Goldman and L. Green, eds.), pp 301-329, CRC Press, Boca Raton, FL).

All microorganisms which can be used for the present invention may be publicly available from different sources, e.g., Deutsche Sammlung von Mikroorganismen und Zellkulturen (DSMZ), Inhoffenstr. 7B, D-38124 Braunschweig, Germany, American Type Culture Collection (ATCC), P.O. Box 1549, Manassas, VA 20108 USA or Culture Collection Division, NITE Biological Resource Center, 2-5-8, Kazusakamatari, Kisarazu-shi, Chiba, 292-0818, Japan (formerly: Institute for Fermentation, Osaka (IFO), 17-85, Juso-honmachi 2-chome,Yodogawa-ku, Osaka 532-8686, Japan).

In connection with the present invention it is understood that the above-mentioned microorganisms also include synonyms or basonyms of such species having the same physiological properties, as defined by the International Code of Nomenclature of Prokaryotes. The nomenclature of the microorganisms as used herein is the one officially accepted (at the filing date of the priority application) by the International Committee on Systematics of Prokaryotes and the Bacteriology and Applied Microbiology Division of the International Union of Microbiological Societies, and published by its official publication vehicle International Journal of Systematic and Evolutionary Microbiology (IJSEM).

The production strain of *Bacillus subtilis,* used for performing the present invention is a genetically engineered one, wherein at least one of the known pantothenate biosynthetic genes has been overexpressed and said strain comprises a deregulation in the biosynthesis of leucine, valine or isoleucine. Preferably, a recombinant *Bacillus subtilis* strain is used. Modifications either on the DNA or protein level include all modification/mutations with a direct impact on the yield, production and/or efficiency of pantothenate production. The person skilled in the art knows how to manipulate a *Bacillus* strain for said purpose. General techniques are described in Harwood, C.R. and Cutting, S.M. (1990). Molecular Biological Methods for Bacillus. Chichester: John Willey and Sambrook, J., Fritsch, E.F. and Maniatis, T. (1989). Molecular Cloning: a Laboratory Manual, 2nd edition. Cold Spring Harbor, NY: Cold Spring Harbor Laboratory. For the scope of the present invention, it is not relevant how the mutant(s) are obtained; such mutants may be obtained, *e.g.,* by site-directed mutagenesis, saturation mutagenesis, random mutagenesis/directed evolution, chemical or UV mutagenesis of entire cells/organisms, and other methods which are known in the art. These mutants may also be generated, *e.g.,* by designing synthetic genes, and/or produced by in vitro (cell-free) translation. For testing of specific activity, these mutants may be (over-) expressed by methods known to those skilled in the art with measurement of the pantothenate production. This and further methods leading to increased pantothenate production by, *e.g.,* the replacement of the natural promoter of the *pan* genes are described in *e.g.* WO 2010/018196. Modification also includes manipulation or deregulation of the branch chain amino acid biosynthesis in the pantothenate production strain.

The term "overexpressing" or "overexpression" means expression of a gene product at a level higher than that expressed prior to modification of the microorganism or in a comparable microorganism which has not been modified. The *Bacillus subtilis* strain may overexpresses one or more genes selected from the group consisting of *panB, panC, panD, panE, ilvB, ilvC, ilvD, ilvN, glyA, serA, serC,* and the *gcv* genes involved in the glycine cleavage pathway; as well as mutants thereof that result in the synthesis of encoded enzymes of improved catalytic properties. Preferably, the recombinant strain used for the purpose of the present invention carries at least two copies of panB and panD (panBD), more preferably 3 or 4 copies leading to increased pantothenate production. The recombinant strain used for the purpose of the present invention is deregulated in the biosynthesis for the branched chain amino acids leucine, valine and/or isoleucine. Said modification is combined with the overexpression of one or more genes selected from the group consisting of *panB, panC, panD, panE, ilvB, ilvC, ilvD, ilvN, glyA, serA, serC,* and the gcv genes involved in the glycine cleavage pathway.

Examples of recombinant strains are described in WO 2007/131750, *i.e.* PA12 (reference strain 1), PA49 (reference strain 2) or PA73 (reference strain 3), or can be generated according to US 5,518,906, US 7,220,561 or WO 2004/005527. Besides the overexpression of the whole panBCDoperon as in reference strains 1-3, the strains may be further modified, such as e.g. deregulation of *glyA* and/or introduction of further copies of *panB* and *panD* (*i.e.* reference strain 4 with *glyA* deregulated and a second copy of *panBD*). These further modification may also include deregulation of the branch chain amino acids biosynthesis (see reference strain 5 as further development of reference strain 4) and/or introduction of further copies of *panBD,* such as, *e.g.,* 2, 3 or 4 copies (see reference strain 6 as further development of reference strain 5). The term "deregulated" or "deregulation" means the alteration or modification of a gene in a microorganism such that the level or activity of the gene product in a microorganism is altered or modified. Preferably, at least one gene is altered or modified such that the gene product is enhanced/increased or attenuated/decreased. With regards to the genes belonging to the pantothenate operon, this deregulation is meant to be an overexpression of at least one of the genes.

The term "genetically engineered" or "genetically altered" means the scientific alteration of the structure of genetic material in a living organism. It involves the production and use of recombinant DNA. More in particular it is used to delineate the genetically engineered or modified organism from the naturally occurring organism. Genetic engineering may be done by a number of techniques known in the art, such as *e.g.* gene replacement, gene amplification, gene disruption, transfection, transformation using plasmids, viruses, or other vectors. A genetically modified organism, *e.g.* genetically modified microorganism, is also often referred to as a recombinant organism, *e.g.* recombinant microorganism.

Using the cultivation medium according to the present invention with a genetically engineered pantothenate-producing strain of *Bacillus subtilis, ,* said strain comprising a deregulation in the biosynthesis of leucine, valine or isoleucine and wherein at least one pantothenate biosynthetic gene is overexpressed, the biosynthesis of pantothenate can be greatly improved. The amount of pantothenate could be increased by at least 10%, preferably by at least 40, 60, 80, 100, 150, 190, 200, 220, 270, 300% compared to cultivation in a medium known in the art (standard medium), *i.e.* a medium wherein the only carbon source is glucose and no raffinose is present, in particular a medium comprising at least 20 g/l glucose. Examples of such media used in the art contain *e.g*. 20 g/l glucose (MMGT), 30 g/l glucose (SVY) or 60 g/l glucose (SVYM and SMG), such as e.g. described in WO 02/057474, WO 02/066665, WO 2004/113510 or WO 2007/131750. The increase is dependent on the degree of engineering level of the production strain. The yield of pantothenate on consumed sugar using a process of the present invention may be in the range of about 12% or more, such as between 12 and 17% or more, in particular about 13, 14, 15, 16, 17, 18, 20% or more.

The present invention provides a process for the production of pantothenate by fermentation. In particular, the present invention provides a process for production of pantothenate comprising converting a substrate into pantothenate. The substrate may be a carbon, nitrogen and phosphate source in which microorganisms are known to utilize to produce biomass, energy, and metabolites, such as pantothenate. A preferred substrate is α-ketoisovalerate. Suitable culture conditions for pantothenate production using *e.g. Bacillus subtilis* are known in the art and described *e.g.* in WO 2004/113510 or WO 2007/065602.

The term "culturing a microorganism under suitable culturing conditions" refers to methods of maintaining and/or growing a living microorganism of the present invention which are well known in the art. The microorganisms can be cultured in liquid, solid or semi-solid media. Preferably, the microorganism is cultured in liquid media comprising nutrients essential or beneficial to the maintenance and/or growth of the microorganism. Such nutrients include, but are not limited to, carbon sources or carbon substrates, such as alcohols, sugars, sugar alcohols, complex carbohydrates such as starches, hydrocarbons, fatty acids, other organic acids, oils, fats; nitrogen sources, *e.g.* vegetable proteins, yeast extract, peptones, peptides and amino acids derived from grains, beans and tubers, or derived from animal sources such as meat or milk, meat extracts and casein hydrolysates; inorganic nitrogen sources such as urea, ammonium sulfate, ammonium chloride, ammonium nitrate and ammonium phosphate; phosphorous sources, *e.g.* phosphoric acid and sodium or potassium salts thereof; trace elements, *e.g.* magnesium, iron, manganese, calcium, copper, zinc, boron, molybdenum and/or cobalt salts; as well as growth factors such as vitamins, growth promoters and the like.

The microorganisms are preferably cultured under controlled pH. In one embodiment, microorganisms are cultured at a pH of between 6.0 and 8.5, more preferably at a pH of about 7, wherein the pH is maintained at a constant level during the whole cultivation period, which is *e.g*. a period of 48 hours. The desired pH may be maintained by any method known to those skilled in the art. Preferably, the microorganisms are further cultured under controlled aeration and under controlled temperatures. In one embodiment, the controlled temperatures include temperatures between 15 and 70°C, preferably the temperatures are between 20 and 55°C, more preferably between 30 and 45°C or between 30 and 50°C.

The microorganisms may be cultured in liquid media either continuously, semi-continuously or batchwise by conventional culturing methods such as standing culture, test tube culture, shaking culture (shake-flask), aeration spinner culture or fermentation. Preferably, the microorganisms are cultured in a fermenter. Fermentation processes of the invention include batch, fed-batch and continuous methods of fermentation. Varieties of such processes have been developed and are well known in the art.

The pantothenate obtained in accordance with the present invention in the fermentation broth can either be used without being recovered or after being recovered. The term "recovering" includes isolating, extracting, harvesting, separating or purifying the compound from the culture medium. Isolating the compound can be performed according to any conventional isolation or purification methodology known in the art including, but not limited to, treatment with a conventional resin, treatment with a conventional adsorbent, alteration of pH, solvent extraction, dialysis, filtration, concentration, crystallization, recrystallization, pH adjustment, and the like. For example, the panthenol compound can be recovered from the culture medium by first removing the microorganisms from the culture. The solution is then passed through or over a cation exchange resin to remove unwanted cations and then through or over an anion exchange resin to remove unwanted inorganic anions and organic acids.

The terms "production" or "productivity" are art-recognized and include the concentration of pantothenate formed within a given time and a given fermentation volume (*e.g.,* kg product per hour per liter). The term "efficiency of production" includes the time required for a particular level of production to be achieved (for example, how long it takes for the cell to attain a particular rate of output of pantothenate). The term "yield" is art-recognized and includes the efficiency of the conversion of the carbon source into the product (*i.e.,* pantothenate). This is generally written as, for example, kg product per kg carbon source. By "increasing the yield and/or production/productivity" of the compound it is meant that the quantity of recovered molecules, or of useful recovered molecules of that compound in a given amount of culture over a given amount of time is increased. The terms "biosynthesis" or a "biosynthetic pathway" are art-recognized and include the synthesis of a compound, preferably an organic compound, by a cell from intermediate compounds in what may be a multistep and highly regulated process.

In one particular aspect, the medium as described herein is useful for screening purpose, *e.g.* for HTP screening of *Bacillus subtilis* strains, which are useful for high production of pantothenate. HTP screening as well as production of pantothenate using the medium under conditions described herein can be performed according to any method known to the skilled person, including *e.g.* the use of shake-flask, deep-well assay or micro-fermentation such as *e.g*. use of a BioLector® (m2p-Labs, 52499 Baesweiler, Germany). BioLector® is a micro-fermentation system allowing HTP screening for optimal fermentation parameters using 48 or 96 well plates with a volume of 100-2000 µl. It performs real-time monitoring of the biomass formation, pH and dissolved oxygen (pO₂). Using these data the optimum B5 production conditions can be determined. Useful protocols for shake-flask assay, micro-fermentation using, *e.g.,* the BioLector® or deep-well assays are known by the skilled person.

A particular useful method for shake-flask assay using strains of *Bacillusin* a production medium according to the invention is as follows: single colonies of *Bacillus* grown on selective TBAB plates or frozen glycerol working cell banks are used to inoculate 10 ml of VY medium (WO 2004/113510) in baffled Erlenmeyer flasks with urethane closures. The culture is grown overnight (about 17 h) at 37°C with an agitation of 300 rpm. 1 ml of overnight cultures are added to 40 ml prewarmed production medium and the cultures are grown at 37°C with an agitation of 300 rpm (Küchner shaker with humidity) in a 250-ml baffled Erlenmeyer flasks with urethane closures. After 24 and 48 h growth, (i) pH is determined, (ii) cell turbidity is measured at 600 nm (OD600nm), and (iii) for determination of sugar, pantothenate and 3-(2-hydroxy-3-methyl-butyrylamino)-propionic acid (HMBPA) side-product content using HPLC assay 2 ml of cultures are centrifuged and 1 ml of supernatants are carefully transferred into Eppendorf tubes. 2 x 150 µl of supernatant per sample are used for HPLC analysis of sugar, B5 and HMBPA as known to the skilled person or as described in the Examples.

A particular useful method for micro-fermentation in a BioLector® using strains of *Bacillus* in a production medium is as follows: Single colonies grown on selective TBAB plates are used to inoculate VY medium at 37°C with an agitation of 250 rpm (Innova 4000 shaker). After 8 h growth the cell turbidity is measured to determine the inoculation volume. The 48-well Flowerplate™ (m2p-Labs, 52499 Baesweiler, Germany) is filled with 1 ml per well of the respective production medium according to the invention and inoculated with the pre-cultures to a starting OD₆₀₀ₙₘ=0.1 in triplicates. The plate is covered with a gas-permeable sealing film that supports the oxygen exchange and a second foil with a perforated sealing film on the top to reduce evaporation. The cultures are grown in the BioLector® at 39°C for 48 h with an agitation of 900 rpm and humidity of 92%, the parameters biomass, pH and pO₂ are measured every 12 min. In addition, after 48 h sugar, B5 and HMBPA contents are determined by HPLC according to known protocols or as described in the Examples.

In one aspect, the medium is used in a process for production of pantothenate or for screening purposes, wherein the medium comprises glucose and raffinose at a ratio of 1:2 to 1:20, and the microorganism is selected from *Bacillus subtilis.*

Advantageous embodiments of the invention become evident from the dependent claims. These and other aspects and embodiments of the present invention should be apparent to those skilled in the art from the teachings herein.

This invention is further illustrated by the following examples which should not be construed as limiting.

### Figures

**Figure 1****.** The 6 different strains were submitted to a 24 deep-well assay according to Example 1. The x-fold increase of pantothenate yield on sugar is shown on the y-axis. The assay was performed using different media, *i.e.* SRG, SRG-C, SRG-Y, and SRG-CY (see Table 1).
**Figure 2****.** The 6 different strains were submitted to a 24 deep-well assay according to Example 1. The x-fold increase of pantothenate yield on sugar is shown on the y-axis. The assay was performed using different media, *i.e.* SRG-FB, SGR-FB-C, SRG-FB-Y, and SRG-FB-CY (see Table 1).

### Examples

As otherwise indicated all strains, culture media as well as fermentation conditions are known in the art and described in more detail in WO 2004/113510 or WO 2007/065602 or WO2004/111214.

### Example 1: Strains and Media

***grains and plasmids.** Bacillus subtilis* strains of the present invention are derived from strain 1A747 (*Bacillus* Genetic Stock Center, The Ohio State University, Columbus, Ohio 43210 USA), which is a prototrophic derivative of *B*. *subtilis* 168 (*trpC2*) (GenBank AL009126). The chloramphenicol-resistance gene (*cat*) cassette was obtained from plasmid pC194 (GenBank M19465, Cat# 1E17 Bacillus Genetic Stock Center, The Ohio State University, Columbus, Ohio 43210 USA). The *S. aureus* erythromycin resistance gene (GenBank V01278) was amplified from plasmid pDG646 (Guérout-Fleury et al., 1995). The *S. aureus* spectinomycin resistance gene (XO3216) was amplified from plasmid pDG1726 (Guérout-Fleury et al., 1995). The *P₁₅* and *P₂₆* promoters of the *B*. *subtilis* bacteriophage SPO1 (Lee et al., 1980, Mol. Gen. Genet. 180:57-65) were obtained from derivatives of plasmid pX12 (Hümbelin et al., 1999, J. Ind. Microbiol. Biotech. 22:1-7) containing the SPO1-15 and SPO1-26 promoters from RB50::[pRF69]::[pRF93] (Perkins et al., 1999, J. Ind. Microbiol. Biotech. 22:8-18). Strains PA12 (Ref 1), PA49 (Ref 2) and PA73 (Ref 3) are described in detail in WO 2007/131750. Strains Ref 4, Ref 5 and Ref 6 are further deregulated derivatives of Ref 3 for high production of pantothenate. This includes deregulation of *glyA* and a second copy of *panBD* for Ref 4, deregulation of *glyA* plus a second copy of *panBD* plus deregulation of the branch chain amino acid synthesis for Ref 5, and deregulation of *glyA* plus 3 or 4 copies of *panBD* plus deregulation of the branch chain amino acid synthesis for Ref 6.

***Production media**.* To test the *B. subtilis* reference strains (Ref 1 to Ref 6) for their B5 production capability 8 different variants of SRG medium were used, including the original SRG. The main characteristics of the media indicating the components that were changed in the SRG medium variants (carbon source, citrate, and YE) are described in Table 1. The amount of glucose FeedBeads (Adolf Kühner AG, 4127 Birsfelden, Schweiz) were selected to get a glucose release of about 10 g/l of glucose after 48 h in the medium. The composition of veal infusion broth-yeast extract medium is described in WO 2004/113510.

The composition of SRG is as follows: 100 ml 10x SMS [20 g (NH₄)₂SO₄, 140 g K₂HPO₄, 60 g KH₂PO₄, 10 g Na₃citrate·H₂O, 2 g MgSO₄·7H₂O, add water to a final volume of 1 l, autoclave (20 min at 121 °C)], 10 ml 100x Trace Elements SMS medium [12.5 g MgCl₂·6H₂O, 0.55 g CaCl₂, 1.35 g FeCl₂·6H₂O, 0.1 g MnCl₂·4H₂O, 0.17 g ZnCl₂, 0.043 g CuCl₂·2H₂O, 0.06 g CoCl₂·6H₂O, 0.06 g Na₂MoO₄·2H₂O, add water to a final volume of 1 l, autoclave (20 min at 121 °C)], 2 ml 50% glucose [50 g glucose add water to a final volume of 100 ml, filter sterilized], 36 ml 25% raffinose [25 g raffinose (D(+)-raffinose), add water to a final volume of 100 ml, filter sterilize], 10 ml 10% yeast extract and 842 ml sterile distilled water. This medium has been also described as RSM in WO2008/148575 (Example 2).

**Table 1: Main characteristics of media tested**

| **Name** | **citrate** | **YE** | **sugar** |
|---|---|---|---|
| SRG | 0.64 g/l | 1 g/l | 1 g/l glucose, 9 g/l raffinose |
| SRG-C | - | 1 g/l | 1 g/l glucose, 9 g/l raffinose |
| SRG-Y | 0.64 g/l | 50 mg/l | 1 g/l glucose, 9 g/l raffinose |
| SRG-CY | - | 50 mg/l | 1 g/l glucose, 9 g/l raffinose |
| SRG-FB | 0.64 g/l | 1 g/l | FeedBeads |
| SRG-FB-C | - | 1 g/l | FeedBeads |
| SRG-FB-Y | 0.64 g/l | 50 mg/l | FeedBeads |
| SRG-FB-CY | - | 50 mg/l | FeedBeads |

***Deep well assay.*** Single colonies grown on selective TBAB plates or frozen glycerol working cell banks were used to inoculate in 24-DW plate containing 3 ml of VY medium per well. The bacteria were grown overnight (about 17 h) at 39°C with an agitation of 550 rpm and humidity of 85 % (Infors HT microtron shaker). Next day a new 24-DW plate was prepared containing 3 ml of production media (SRG medium or a SRG variant) per well. For inoculation 30 µl of overnight cultures were used. Plates were covered with a gas-permeable sealing film that supports the oxygen exchange. The cultures grew for 48 h in Infors HT microtron shaker as described above. After 48 h growth, samples were taken for determining the sugar, B5 and HMBPA content by HPLC and the cell turbidity at 600 nm (OD600nm).

***HPLC analytics for vitamin B5 and HMBPA*** Chromatography of samples was performed on a Phenomenex LUNA C8 column, using an Agilent 1100 HPLC system equipped with a thermostatted autosampler and a diode array detector. The column dimensions are 150 x 4.6 mm, particle size 5 micron. The column temperature was kept constant at 20°C. The mobile phase is a mixture of 0.1% acetic acid (A) and methanol (B). Gradient elution is applied, ranging from 1% B to 45% B in 15 minutes. The flow rate is 1 ml/min. Pantothenate was monitored using UV absorption at 220 nm, and is eluted at approximately 9.6 min. The calibration range of the method is from 1 to 100 mg/l pantothenate. Other pantothenate related metabolites were also separated and the concentration was determined using this method.

***HPLC assay for the analysis of sugars.*** The concentration of glucose and raffinose in the culture broth was analyzed by an Agilent 1100 series HPLC system using a quaternary pump, an autosampler a UV- and a refractive index detector. The separation was achieved on a CAPCELL PAK NH2 UG80 column (4.6 mm x 250 mm, 5µ) (Shiseido). The optimal column temperature was 35°C. The mobile phase was a mixture of acetonitrile and DI water at a 65/35 ratio. The flow rate was 1.0 ml/min and the injection volume set to 5 µl. The refractive index signal was monitored and used for detection. The calibration range for each compound is from 0.5 mg/ml to 30 mg/ml.

### Example 2: Production of vitamin B5 in 24 deep-well experiments: influence of citrate and YE

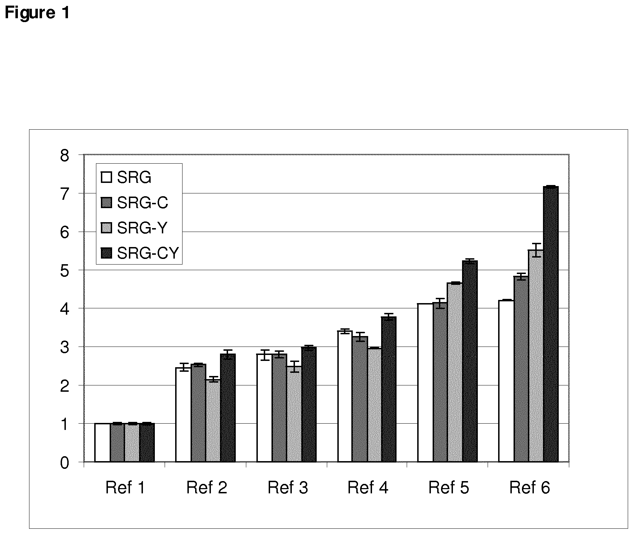

The six reference strains (Ref 1 to Ref 6) were tested in HTP 24-DW plate assay using 4 media: SRG, SRG-C, SRG-Y and SRG-CY (see Table 1). In the SRG medium strain Ref 6 gave 4.2-fold higher B5 yield on sugar than strain Ref 1 (Fig. 1) and all the sugar was consumed after 48 h. In the SRG-C medium strain Ref 6 gave 4.8-fold higher B5 yield on sugar than strain Ref 1 and all the sugar was consumed. In SRG-Y medium and SRG-CY medium only strain Ref 5 and Ref 6 consumed all the sugar while strains Ref 1 to Ref 4 consumed only 60-80 % of the carbon source at 48 h. Therefore, the B5 yields were calculated on the consumed sugar. Accordingly, in SRG-Y medium strain Ref 6 showed 5.5-fold increase of B5 yield on consumed sugar compared to strain Ref 1 while in SRG-CY medium the increase of B5 yield on sugar was 7.2 fold between strain Ref 1 and strain Ref 6 (Fig. 1). The results demonstrated that the presence of citrate and/or the decrease in YE did not significantly influence the B5 production ranking (capabilities) of the six *B*. *subtilis* B5 reference strains. Lowering of the yeast extract to 50 mg/l leads to an incomplete sugar consumption of the strains Ref 1 to Ref 4.

### Example 3: Production of vitamin B5 in 24 deep-well experiments: influence of sugar source

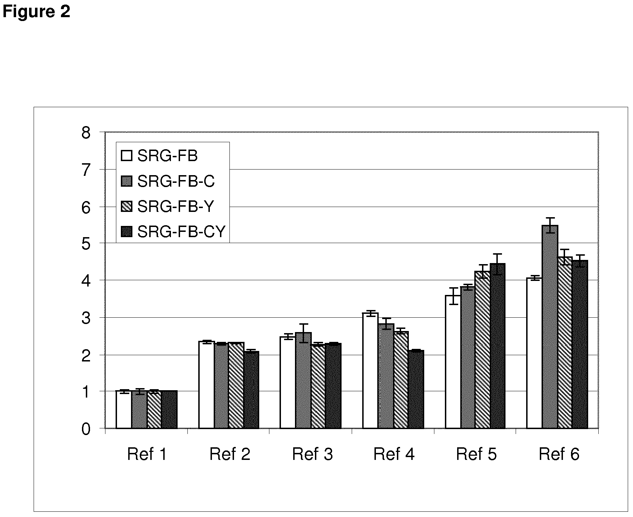

Next, the HTP screening B5 production assay was performed with slow release glucose FeedBeads instead of the use of raffinose: the six *B*. *subtilis* B5 reference strains were tested in HTP 24-DW plate assay using 4 media: SRG-FB, SRG-FB-C, SRG-FB-Y and SRG-FB-CY (see Table 1). In the SRG-FB medium strain Ref 6 gave 4.1-fold higher B5 yield on sugar than strain Ref 1 (Fig. 2) and all the sugar was consumed after 48 h. This is comparable to the results with SRG medium, but the absolute B5 yield on sugar decreased approximately 25% compared to SRG medium. In the SRG-FB-C medium strain Ref 6 gave 5.5-fold higher B5 yield on sugar than strain Ref 1 and all the sugar was consumed, but the B5 yield on sugar decreased compared to SRG-FB medium. In SRG-FB-CY strain Ref 6 gave 4.6-fold higher B5 yield on sugar than strain Ref 1 (Fig. 2) and all the sugar was consumed. The higher engineered strains showed an increase of B5 yield on sugar of 20% higher compared to SRG-FB. In SRG-FB-CY strain Ref 6 gave 4.5-fold higher B5 yield on sugar than strain Ref 1 (Fig. 2) and all the sugar was consumed.

In SRG-FB-Y and SRG-FB-CY only slight differences between the production capability of the less engineered strains were visible. If the raffinose is exchanged to slow release glucose FeedBeads, the sugar is completely consumed when the yeast extract is lowered to 50 mg/l, but the B5 yield on sugar differences between the strains Ref 1 to Ref 4 were only slightly visible in SRG-FB-Y and SRG-FB-CY.

## Claims

1. A process for the production of pantothenate using a genetically engineered pantothenate-producing strain of *Bacillus subtilis,* said strain comprising a deregulation in the biosynthesis of leucine, valine and/or isoleucine and wherein at least one pantothenate biosynthetic gene is overexpressed, said process comprising cultivation in a medium comprising 10g/l or less of sugar supplemented with 50 mg/l yeast extract, wherein 100% of the sugar is consumed after 48 hours of cultivation period.

2. A process according to claim 1, wherein the medium is supplemented with 0.64 g/l citrate.

3. A process according to claim 1 or 2, wherein the sugar is a mixture of glucose and raffinose.

4. A process according to any one of claims 1 or 3, wherein the pH is maintained at a constant level during 48 hours of cultivation period.

5. A process according to any one of claims 1 to 4, wherein the pantothenate is recovered from the medium.

6. A process according to any one of claims 1 to 5, wherein the microorganism comprises at least 2 copies of *panBD.*

7. A process according to any one of claims 1 to 6, wherein at least one of the genes encoding enzymes PanC, PanE and IlvD is overexpressed within the microorganism.

8. A process according to any one of claims 1 to 7, wherein the microorganism is deregulated in glyA.

9. A process according to any one of claims 1 to 8, wherein the sugar is released from polymer particles.

10. Use of a process according to any one of claims 1 to 9 for increasing the amount of pantothenate by at least 10% compared to a process using a standard medium comprising at least 20 g/l glucose.

## Patentansprüche

1. Verfahren zur Herstellung von Pantothenat unter Verwendung eines genetisch modifizierten pantothenatproduzierenden Stamms von *Bacillus subtilis,* wobei der Stamm eine Deregulierung in der Biosynthese von Leucin, Valin und/oder Isoleucin umfasst und wobei mindestens ein Pantothenatbiosynthesegen überexprimiert wird, wobei das Verfahren die Kultivierung in einem 10 g/l oder weniger Zucker ergänzt mit 50 mg/l Hefeextrakt umfassenden Medium umfasst, wobei nach einer Kultivierungsdauer von 48 Stunden 100% des Zuckers verbraucht ist.

2. Verfahren nach Anspruch 1, wobei das Medium mit 0,64 g/l Citrat ergänzt ist.

3. Verfahren nach Anspruch 1 oder 2, wobei es sich bei dem Zucker um eine Mischung von Glucose und Raffinose handelt.

4. Verfahren nach einem der Ansprüche 1 oder 3, wobei der pH-Wert während der 48stündigen Kultivierungsdauer konstant gehalten wird.

5. Verfahren nach einem der Ansprüche 1 bis 4, wobei das Pantothenat aus dem Medium gewonnen wird.

6. Verfahren nach einem der Ansprüche 1 bis 5, wobei der Mikroorganismus mindestens zwei Kopien von *panBD* umfasst.

7. Verfahren nach einem der Ansprüche 1 bis 6, wobei wenigstens eines der Gene, die die Enzyme PanC, PanE und IlvD codieren, im Mikroorganismus überexprimiert wird.

8. Verfahren nach einem der Ansprüche 1 bis 7, wobei der Mikroorganismus in glyA dereguliert ist.

9. Verfahren nach einem der Ansprüche 1 bis 8, wobei der Zucker aus Polymerpartikeln freigesetzt wird.

10. Verwendung eines Verfahrens nach einem der Ansprüche 1 bis 9 zum Erhöhen der Menge an Pantothenat um mindestens 10% im Vergleich zu einem Verfahren, bei dem ein mindestens 20 g/l Glucose umfassendes Standardmedium verwendet wird.

## Revendications

1. Procédé de production de pantothénate au moyen d'une souche productrice de pantothénate génétiquement modifiée de *Bacillus subtilis,* ladite souche comprenant une dérégulation de la biosynthèse de leucine, de valine et/ou d'isoleucine et dans laquelle au moins un gène de biosynthèse de pantothénate est surexprimé, ledit procédé comprenant une culture dans une milieu comprenant 10 g/l ou moins de sucre supplémenté avec 50 mg/l d'extrait de levure, dans lequel 100 % du sucre est consommé après 48 heures de période de culture.

2. Procédé selon la revendication 1, dans lequel le milieu est supplémenté avec 0,64 g/l de citrate.

3. Procédé selon la revendication 1 ou 2, dans lequel le sucre est un mélange de glucose et de raffinose.

4. Procédé selon l'une quelconque des revendications 1 ou 3, dans lequel le pH est maintenu à un niveau constant pendant une période de culture de 48 heures.

5. Procédé selon l'une quelconque des revendications 1 à 4, dans lequel le pantothénate est récupéré à partir du milieu.

6. Procédé selon l'une quelconque des revendications 1 à 5, dans lequel le micro-organisme comprend au moins 2 copies de *panBD.*

7. Procédé selon l'une quelconque des revendications 1 à 6, dans lequel au moins un des gènes codant pour les enzymes PanC, PanE et IlvD est surexprimé dans le micro-organisme.

8. Procédé selon l'une quelconque des revendications 1 à 7, dans lequel le micro-organisme est dérégulé dans glyA.

9. Procédé selon l'une quelconque des revendications 1 à 8, dans lequel le sucre est libéré à partir des particules de polymère.

10. Utilisation d'un procédé selon l'une quelconque des revendications 1 à 9 pour augmenter la quantité de pantothénate d'au moins 10 % par rapport à un procédé utilisant un milieu standard comprenant au moins 20 g/l de glucose.
